(19) Europäisches Patentamt
European Patent Office
Office européen des brevets

(11) Veröffentlichungsnummer: **0 133 164**
**B1**

(12) # EUROPÄISCHE PATENTSCHRIFT

(45) Veröffentlichungstag der Patentschrift: **14.12.88**

(51) Int. Cl.⁴: **C 07 D 249/20**, C 08 K 5/34

(21) Anmeldenummer: **84810357.8**

(22) Anmeldetag: **20.07.84**

(54) **Copolymerisierbare Verbindungen.**

(30) Priorität: **26.07.83 CH 4067/83**

(43) Veröffentlichungstag der Anmeldung: **13.02.85 Patentblatt 85/7**

(45) Bekanntmachung des Hinweises auf die Patenterteilung: **14.12.88 Patentblatt 88/50**

(84) Benannte Vertragsstaaten: **DE FR GB IT NL**

(56) Entgegenhaltungen:
**EP-A-0 031 302**
**EP-A-0 057 160**
**DE-B-1 217 383**

(73) Patentinhaber: **CIBA- GEIGY AG, Klybeckstrasse 141, CH- 4002 Basel (CH)**

(72) Erfinder: **Slongo, Mario, Dr., Sägetrainweg 553, CH- 1712 Tafers (CH)**
Erfinder: **Rody, Jean, Dr., Rütiring 82, CH- 4125 Riehen (CH)**

**EP 0 133 164 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1988

**Beschreibung**

Die vorliegende Erfindung betrifft neue copolymerisierbare 2-(2'-Hydroxyphenyl)-benztriazole, deren Verwendung zum Schützen von Polymerisaten gegen lichtinduzierten Abbau, sowie die damit ausgerüsteten Polymeren.

Es ist bekannt, ethylenisch ungesättigte Derivate von Benztriazolen polymerisierbaren Monomeren zuzugeben und diese dann zu einem gegen UV-Licht geschützten Copolymeren reagieren zu lassen. Solche einbaubaren Stabilisatoren sind z. B. in der US-A-3 399 173 beschrieben. Bekannte copolymerisierbare Additive sind jedoch zuwenig im Co-monomeren löslich, was zur Störung der Copolymerisation führt, oder sie sind z.T. nur schwer zugänglich. Ausserdem werden in der EP-A-57 160 Benztriazol-UV-Absorber beschrieben, welche sich u.a. für Lacksysteme, wie Automobillacke eignen. Die erfindungsgemässen Verbindungen besitzen diese Nachteile nicht oder in geringerem Ausmass und besitzen darüber hinaus den Vorteil hoher Migrationsechtheit und der Möglichkeit, hohe Konzentrationen an UV-Absorber in dünnen Schichten, wie Schutzlacken, einzusetzen. Dabei ist es wichtig, dass sich die übrigen Eigenschaften des zu schützenden Polymers nicht oder nur unwesentlich ändern.

Aus der DE-B-1 217 383 sind 2-(2-Hydroxyphenyl)-benztriazole bekannt, die wie die erfindungsgemässen Verbindungen in 5-Stellung des Phenylringes einen

$-(CH_2)_m\overset{\overset{\displaystyle O}{\|}}{C}X$-Rest

(Definitionen siehe unten) tragen können. Die aus dieser DE-B bekannten Verbindungen weisen jedoch keine polymerisierbare Doppelbindungen enthaltende Substituenten auf und können daher, im Gegensatz zu den erfindungsgemässen Verbindungen, nicht in zu schützende Substrate einpolymerisiert werden.

Die vorliegende Erfindung betrifft Verbindungen der Formel I

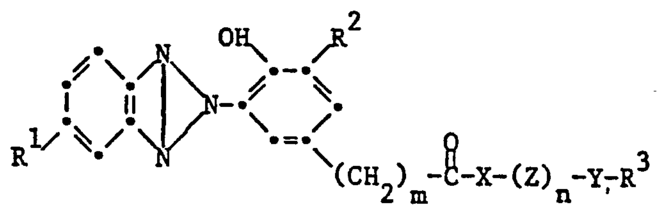

(I)

worin

- X $-O-$ oder $-N(R^4)-$ bedeutet, und
- Y $-O-$ oder $-N(R^5)-$ ist, und
- Z $C_2$-$C_{12}$ Alkylen, durch ein bis drei $-N(R^4)$-Gruppen und/oder Sauerstoff-Atome unterbrochenes $C_4$-$C_{12}$ Alkylen, durch eine Hydroxygruppe substituiertes $C_3$-$C_{12}$ Alkylen, Butenylen, Butinylen, Cyclohexylen oder Phenylen bedeutet, und
- m eine Zahl 0, 1 oder 2 ist, und
- n 1, und falls X und Y $-N(R^4)$- bzw. $-N(R^5)$ sind, auch 0 bedeutet, und
- $R^1$ Wasserstoff, Chlor, $C_1$-$C_4$ Alkyl oder $C_1$-$C_4$ Alkoxy ist, und
- $R^2$ Wasserstoff oder $C_1$-$C_8$ Alkyl bedeutet, und
- $R^3$ eine Gruppe $-C(O)-C(R^6) = C(H)R^7$ ist, oder, falls Y $-N(R^5)$- ist, zusammen mit $R^5$ eine Gruppe $-C(O)-CH{=}CH-C(O)-$ bildet, wobei $R^6$ Wasserstoff oder Methyl ist, und $R^7$ Wasserstoff, Methyl oder $-C(O)-X-R^8$ bedeutet, wobei $R^8$ Wasserstoff, $C_1$-$C_{12}$ Alkyl oder eine Gruppe der Formel II bedeutet

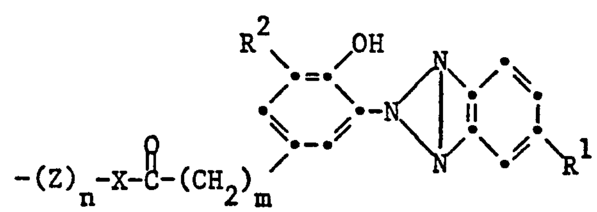

(II)

worin die Symbole $R^1$, $R^2$, X, Z, m and n die oben angegebene Bedeutung haben, und $R^4$ und $R^5$ unabhängig voneinander Wasserstoff, $C_1$-$C_{12}$ Alkyl, durch ein bis 3 Sauerstoffatome unterbrochenes $C_3$-$C_{12}$ Alkyl, Cyclohexyl oder $C_7$-$C_{11}$ Aralkyl bedeuten, und $R^4$ ferner zusammen mit $R^5$, falls Z Ethylen ist auch Ethylen bilden.

X und Y können unabhängig voneinander -O- oder $-N(R^4)$- bzw. $-N(R^5)$-sein. Hervorzuheben sind Verbindungen, in welchen eines von X und Y -O- ist, und insbesondere solche, in welchen sowohl X wie auch Y -O- ist.

Z ist als $C_2$-$C_{12}$ Alkylen geradkettig oder verzweigt. Dabei handelt es sich z. B. um Ethylen, Propylen, Tetramethylen, Hexamethylen, Octamethylen, Dodecamethylen, 1,1-Ethyliden, 2,2-Propyliden, 2-Methylpentamethylen oder 2-Ethylhexamethylen. Bevorzugt sind $C_2$-$C_6$ Alkylengruppen.

Bedeutet Z $C_4$-$C_{12}$ Alkylen, welches durch Sauerstoff unterbrochen ist, so handelt es sich z. B. um $-CH_2-CH_2-O-CH_2-CH_2-$, $-CH_2-CH_2-O-CH_2-CH_2-CH_2$, $CH_2-CH_2-O-CH_2-CH_2-O-CH_2-CH_2$, $-CH_2-CH_2-O-CH_2-CH_2-O-CH_2-CH_2-O-CH_2-CH_2-$ und Alkylen, das durch Gruppen $-N(R^4)-$ unterbrochen ist, bedeutet beispielsweise $-CH_2-CH_2-NH-CH_2-CH_2-CH_2-CH_2-$, $-CH_2-CH_2-CH_2-NH(CH_2)_8-$ oder $-CH_2-CH_2-CH_2-N(CH_3)-CH_2-CH(C_2H_5)(CH_2)_4-$.

Z als durch eine Hydroxygruppe substituiertes $C_3$-$C_{12}$-Alkylen ist 2-Hydroxytetramethylen, 2-Hydroxyhexamethylen und insbesondere 2-Hydroxypropylen.

Z ist als Cyclohexylen z. B. Cyclohexylen-1,4 und insbesondere Cyclohexylen-1,2.

Z ist als Phenylen z. B. Phenylen-1,3 oder Phenylen-1,4.

m kann 0, 1 oder 2 sein. Bevorzugt ist m 2.

n ist bevorzugt 1, kann aber auch 0 sein, falls sowohl X als auch Y über Stickstoff gebunden sind.

$R^1$ als $C_1$-$C_4$ Alkyl ist beispielsweise Methyl, Ethyl, n-Propyl, Isopropyl, n-Butyl, sec. Butyl oder tert. Butyl.

Bedeutet $R^1$ $C_1$-$C_4$ Alkoxy, so handelt es sich z. B. um Methoxy, Ethoxy, n-Propoxy, Isopropoxy, n-Butoxy oder tert. Butoxy.

Bevorzugte Bedeutung von $R^1$ ist Wasserstoff oder Chlor.

$R^2$ als $C_1$-$C_8$ Alkyl ist beispielsweise Methyl, Ethyl, n-Propyl, Isopropyl, n-Butyl, sec. Butyl, tert. Butyl, n-Pentyl, tert. Amyl, n-Hexyl, n-Heptyl, n-Octyl, 2-Ethylhexyl oder 1,1,3,3-Tetramethylbutyl. Bevorzugt ist tert. Butyl.

$R^4$, $R^5$ und $R^8$ als $C_1$-$C_{12}$ Alkyl können die gleiche oben für $R^2$ angegebene Bedeutung haben und zusätzlich geradkettiges oder verzweigtes Nonyl, Decyl, Undecyl oder Dodecyl sein.

Sind $R^4$ und $R^5$ durch Sauerstoff-Atome unterbrochenes Alkyl, so sind dieselben Beispiele, wie oben für Z beschrieben, zu nennen.

Beispiele für $R^4$ und $R^5$ als Aralkyl sind Benzyl, α-Methylbenzyl, 1-Phenylethyl, α,α-Dimethylbenzyl, oder 1-Phenylpropyl.

Falls Z Ethylen ist, können $R^4$ und $R^5$ zusammen ebenfalls Ethylen bilden, was einer Überbrückung über einen Piperazinrest gleichkommt.

ist Y eine Gruppe $-N(R^5)-$, so kann $R^3$ und $R^5$ zusammen eine Gruppe $-C(O)-CH=CH-C(O)-$ bilden was eine Substitution des Rests

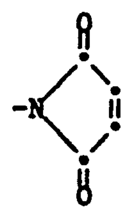

an die Gruppe $-X-(Z)_n-$ bedeutet.

Die bevorzugte Bedeutung von $R^3$ ist allerdings $-C(O)-C(R^6)=C(H)R^7$.

$R^6$ und $R^7$ sind bevorzugt Methyl und insbesondere Wasserstoff.

Besonders bevorzugt sind Verbindungen der Formel I, worin X -O- oder $-N(R^4)-$ und Y -O- oder $-N(R^5)-$ bedeutet, Z $C_2$-$C_6$ Alkylen, 2-Hydroxypropylen oder Cyclohexylen ist, m 2 und n 1 bedeutet, und $R^1$ Wasserstoff oder Chlor ist, und $R^2$ Wasserstoff oder $C_1$-$C_8$ Alkyl bedeutet und $R^3$ eine Gruppe $-C(O)-C(R^6)=C(H)R^7$ ist, wobei $R^6$ und $R^7$ unabhängig voneinander Wasserstoff oder Methyl sind, und $R^3$, falls Y $-N(R^5)-$ ist, zusammen mit $R^5$ eine Gruppe $-C(O)-CH=CH-C(O)-$ bildet, und $R^4$ und $R^5$ unabhängig voneinander Wasserstoff oder $C_1$-$C_4$ Alkyl sind.

Beispiele für Verbindungen der Formel I sind solche, die der Formel II entsprechen:

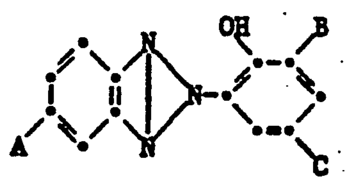

(II)

| A | B | C |
|---|---|---|
| H oder Cl | -tert. $C_4H_9$ | $-CH_2CH_2C(O)NH-CH_2CH_2OC(O)CH=CH_2$ |
| H oder Cl | -tert. $C_4H_9$ | $-CH_2CH_2C(O)-$ 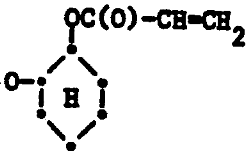 |
| H oder Cl | -tert. $C_5H_{11}$ | $-CH_2CH_2C(O)NHCH_2CH_2-$ 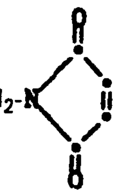 |
| H oder Cl | $-C(CH_3)_2CH_2C(CH_3)_3$ | $-CH_2CH_2C(O)NHNHC(O)CH=CH_2$ |
| H oder Cl | -sec. $C_4H_9$ | $-CH_2CH_2C(O)OCH_2CH_2NHC(O)C(CH_3)=CH_2$ |
| $-CH_3$ | -tert. Butyl | $-CH_2CH_2C(O)OCH_2CH_2OC(O)C(CH_3)=CH_2$ |
| $-OCH_3$ | -tert. $C_5H_{11}$ | $-C(O)NH-(CH_2)_6NHC(O)CH=CH_2$ |
| H oder Cl | -tert. $C_4H_9$ | $-CH_2CH_2C(O)O-CH_2CH_2OCH_2CH_2-OC(O)CH=CH_2$ |
| H oder Cl | -tert. $C_5H_{11}$ | $-CH_2CH_2C(O)O-CH_2CH_2CH_2CH_2OC(O)CH=C(H)CH_3$ |
| H oder Cl | -tert. $C_4H_9$ | $-CH_2CH_2C(O)N(CH_3)CH_2CH_2N(CH_3)C(O)CH=CH_2$ |
| $CH_3$ | -iso-$C_3H_7$ | $-CH_2CH_2C(O)OCH_2CH_2N(C_4H_9)C(O)C(CH_3)=CH_2$ |
| H oder Cl | -tert. $C_4H_9$ | $-CH_2CH_2C(O)-$ 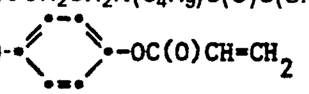 |
| H oder Cl | -tert. $C_4H_9$ | $-CH_2CH_2C(O)N$ 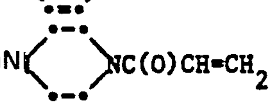 |
| $-OCH_3$ | -tert. $C_4H_9$ | $-CH_2C(O)NHCH_2CH_2OC(O)C(CH_3)=C(H)CH_3$ |
| H oder Cl | -tert.$C_4H_9$ | $-CH_2CH_2C(O)O-$ 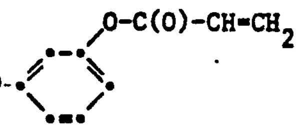 |
| H oder Cl | -tert $C_5H_{11}$ | $-CH_2CH_2C(O)NHCH_2CH_2NHCH_2CH_2NHC(O)CH=CH_2$ |

Die Herstellung der Verbindungen der Formel I erfolgt auf an sich bekannte Weise. So wird z. B. ein gegebenenfalls substituiertes o-Nitroanilin diazotiert und mit einer Verbindung der Formel III

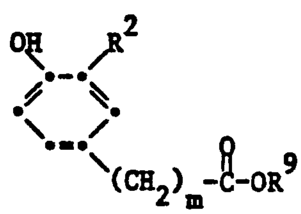

(III)

bei einem pH von ungefähr 8 bis 11 gekuppelt. Die Symbole $R^2$ und m sind dabei wie oben angegeben definiert und $R^9$ bedeutet Wasserstoff oder Niederalkyl. Die Reaktion verläuft nach folgender Gleichung:

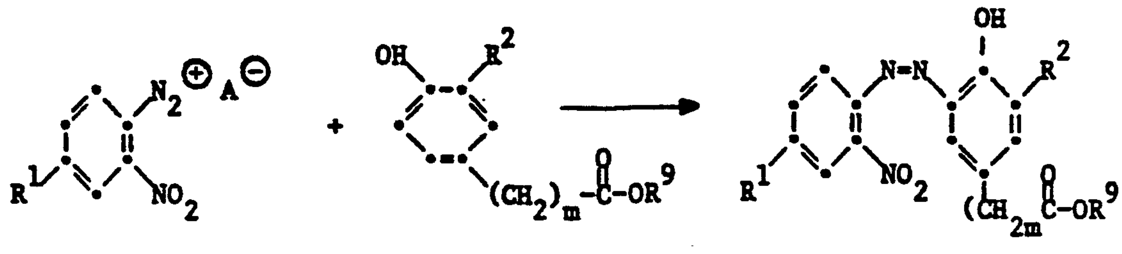

X bedeutet dabei ein Anion wie $Cl^{\ominus}$, $-HSO_4^{\ominus}$ oder $(BF_4)^{\ominus}$. Aus dem z. B. wie oben beschrieben hergestellten Produkt wird durch reduzierende Triazolierung unter alkalischen Bedingungen das entsprechende Benztriazol-Derivat hergestellt.

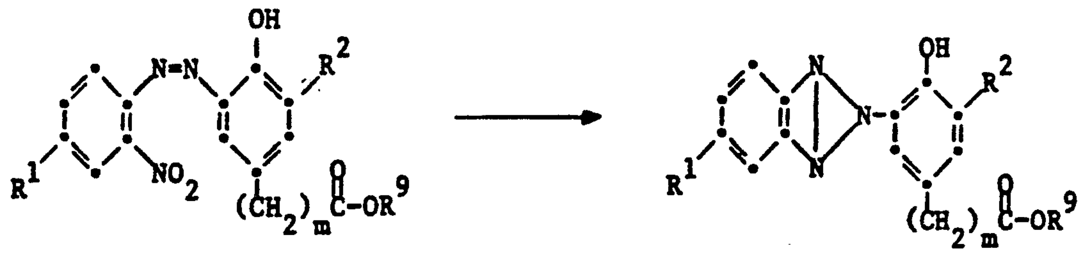

Bei der reduktiven Triazolierung erfolgt die Reaktion vorzugsweise katalytisch durch Wasserstoff oder es wird z. B. Zinkstaub als Reduktionsmittel eingesetzt. Durch das alkalische Milieu wird zwar der Ester ($R^9$) verseift, er kann jedoch nach dem Ansäuern leicht durch Kochen in einem Niederalkylalkohol, wie Methanol wieder hergestellt werden.

Das so gewonnene Produkt ist Zwischenprodukt, um z. B. mit Verbindungen der Formel $HX-(Z)_n-YH$ umgesetzt zu werden. Dabei handelt es sich um eine übliche Umesterung, welche zu Verbindungen der Formel

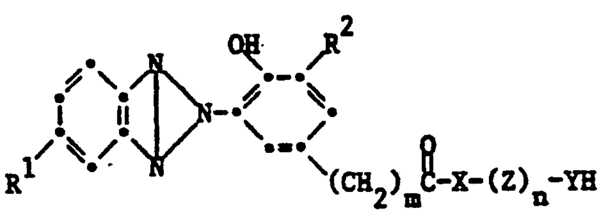

führt. Letztere können auf bekannte Weise mit Chlorpropionsäurechlorid zu den Verbindungen der Formel I verestert werden. Die verschiedenen Verfahrensschritte sind dem Fachmann bekannt und im Detail in der EP-A-57 160 beschrieben.

Die erfindungsgemäss verwendbaren 2-(2'-Hydroxyphenyl)-benztriazolverbindungen können zwischen 0,01 bis 20 % der polymerisierbaren Mischung ausmachen. Wird das entstehende Copolymer direkt zu Artikeln mit Dicken über 0,1 mm verarbeitet, so sind Gehalte von 0,05 bis 2 % bezüglich des Gesamtgewichtes bevorzugt, bei Polymeren, die als sehr dünne Überzüge (sogenannte coatings) verwendet werden, sowie bei Polymeren, die als Stammkonzentrate konventionell hergestellten Polymeren einverleibt werden sollen, sind Gehalte von 2 - 20 % bevorzugt. In diesem Falle sind die farblosen Benztriazolverbindungen besonders wertvoll, da sich bei solch hohen Konzentrationen sonst leicht starke Gelbfärbungen ergeben.

Als Beispiele für ethylenisch ungesättigte Verbindungen, die für die Herstellung der erfindungsgemässen Copolymeren in Frage kommen, seien folgende genannt:

1. monosubstituierte Ethylene, das heisst Vinylverbindungen, wie Vinylhalogenide, zum Beispiel Vinylfluorid oder Vinylchlorid; Vinylester organischer Carbonsäuren, zum Beispiel Vinylacetat, Vinylstearat oder Vinylbenzoat; Vinylether, zum Beispiel Vinylisopropylether oder Vinylphenylether; Vinylketone, beispielsweise Vinylmethylketon oder Vinylcyclohexylketon; N-Vinyl-Verbindungen, zum Beispiel Vinylpyrrolidon, Vinylcarbazol oder Vinylisocyanat; S-Vinylverbindungen, wie Vinylmethylthioether, Vinylmethylsulfon oder Vinylsulfonsäure; vinylsubstituierte homocyclisch- oder heterocyclisch-aromatische Verbindungen, wie Styrol, Vinyltoluol, 1-Vinylnaphthalin oder 2-Vinylpyridin; Acrylverbidungen, beispielsweise Acrylsäure, Acrylnitril, Acrylsäurephenylester, Acrylsäureethylester, Acrylsäureamid oder Acrylsäurebutylamid; Allylverbindungen, wie Ester und Ether des Allylalkohols, zum Beispiel Allylbenzoat oder Phenylallylether; Derivate des Allylamins, wie Essigsäureallylamid, Allylharnstoff oder N-Allyl-2,4,6-triamino-1,3,5-triazin oder C-Allylverbindungen wie Allylbenzol;

2. 1,1-disubstituierte Ethylene, das heisst Vinylidenverbindungen, beispielsweise Vinylidenchlorid, Vinylidencyanid, Methacrylnitril, Methacrylsäuremethylester, α-Chloracrylsäureethylester, α-Methylstyrol,

Isobutylen oder Isopropenylacetat;

3. 1,2-disubstituierte Ethylene, wie Vinylencarbonat, Maleinsäureanhydrid, Maleinsäureimid, Fumarsäureethylester, Maleinsäuredinitril, Acenaphthylen oder S,S-dioxobenzthiophen;

4. Di- und Polyene, besonders konjugierte Polyene, wie Butadien, Isopren, Chloropren, Sorbinsäure, Sorbinsäuremethylester, oder Verbindungen mit mehreren isolierten Doppelbindungen, wie Divinylbenzol, Acrylsäureallylester, Phthalsäurediallylester, Glukosetriallylether oder N,N',N''-Triallyl-2,4,6-triamino-1,3,5-triazin.

Die homopolymerisierbaren der genannten Monomeren können zusammen mit den erfindungsgemässen Benztriazolmonomeren copolymerisiert oder aber mit weiteren Monomeren zu Ter- und Quaterpolymeren umgesetzt werden. Das Molekulargewicht der erhaltenen Polymere ist, sofern die zur Erreichung der üblichen und notwendigen mechanischen und elektrischen Eigenschaften nötigen minimalen Grenzwerte überschritten werden, von geringer Bedeutung. Je nach Polymeren soll es zwischen 5000 und mehreren Millionen liegen.

Die Copolymerisation der ethylenisch ungesättigten Verbindungen mit den 2-(2'-Hydroxyphenyl)-benztriazolverbindungen, welche mindestens eine copolymerisierbare ethylenisch ungesättigte Gruppe enthalten, erfolgt auf übliche Weise. Sie wird zum Beispiel mittels freier Radikale ausgelöst, wie sie beispielsweise beim Erwärmen von Benzoylperoxyd, α,α'-Azodiisobutyronitril und tert.-Butylperoxyd oder in Redoxsystemen, wie Mischungen von Salzen der Perschwefelsäure und der schwefligen Säure oder durch Einwirkung energiereicher Strahlungen entstehen. In manchen Fällen gelingen auch ionische Polymerisationen; doch sind diese wegen des stark polaren Charakters der monomeren 2-(2'-Hydroxyphenyl)-benztriazolverbindungen schwierig durchzuführen.

Die Verarbeitung der erfindungsgemässen Polymeren erfolgt auf üblichem Weg, zum Beispiel durch Verspritzen, Pressen, Kalandrieren und Giessen. Die Polymeren können auch noch polymerhomolog weiter umgesetzt werden, zum Beispiel können Polyvinylester unter Bildung von Polyvinylalkoholen verseift werden.

Die anfänglichen physikalischen Eigenschaften der neuen Additionspolymeren sind (mit Ausnahme der UV-Absorption) denjenigen der konventionell, das heisst in Abwesenheit von einpolymerisierbaren 2-(2'-Hydroxyphenyl)-benztriazolverbindungen hergestellten Polymeren sehr ähnlich. Die Verarbeitung kann daher im allgemeinen in beiden Fällen auf die gleiche Art und Weise erfolgen. Bei der Alterung unter Lichteinfluss zeigen erfindungsgemässe Additionspolymere jedoch eine wesentlich geringere Veränderung der ursprünglichen physikalischen Eigenschaften und sind auch viel weniger den sonst üblichen Verfärbungen unterworfen.

Im allgemeinen finden erfindungsgemässe Copolymere gleiche Anwendung wie entsprechende, konventionell hergestellte Polymere. Zusätzliche Anwendungen ergeben sich für erfindungsgemässe Copolymere dank ihrer Lichtstabilität und ihrem UV-Absorptionsvermögen, sei es als Konstruktionsmaterial, Beschichtungsmaterial oder als UV-Filter in der Verpackungsindustrie.

Zusammen mit den erfindungsgemässen Stabilisatoren können auch andere Additive zugegeben werden, beispielsweise Antioxidantien, andere UV-Absorber, Metallcarboxylate, Gleitmittel, Antistatika, Flammschutzmittel, Pigmente, Füll- oder Verstärkungsstoffe. Ausserdem kann man Vernetzungshilfsmittel zusetzen, wie z. B. Triallylcyanurat, Diallylterephthalat, Triallyltrimethithat, Ethylenglykol-diacrylat oder Trimethylalpropan-trimethacrylat.

Untenstehend sind Beispiele weiterer Zusätze, mit welchen die erfindungsgemässen Benztriazole eingesetzt werden können, aufgeführt:

**1. Antioxidantien**

<u>1.1. Alkylierte Monophenole</u>
2,6-Di-tert.butyl-4-methylphenol
2-Tert.butyl-4,6-dimethylphenol
2,6-Di-tert.butyl-4-ethylphenol
2,6-Di-tert.butyl-4-n-butylphenol
2,6-Di-tert.butyl-4-i-butylphenol
2,6-Di-cyclopentyl-4-methylphenol
2-(α-Methylcyclohexyl)-4,6-dimethylphenol
2,6-Di-octadecyl-4-methylphenol
2,4,6-Tri-cyclohexylphenol
2,6-Di-tert.butyl-4-methoxymethylphenol

<u>1.2. Alkylierte Hydrochinone</u>
2,6-Di-tert.butyl-4-methoxyphenol
2,5-Di-tert.butyl-hydrochinon
2,5-Di-tert.amyl-hydrochinon
2,6-Diphenyl-4-octadecyloxyphenol

1.3. Hydroxylierte Thiodiphenylether

2,2'-Thio-bis-(6-tert.butyl-4-methylphenol)
2,2'-Thio-bis-(4-octylphenol)
4,4'-Thio-bis-(6-tert.butyl-3-methylphenol)
4,4'-Thio-bis-(6-tert.butyl-2-methylphenol)

1.4. Alkyliden-Bisphenole

2,2'-Methylen-bis-(6-tert.butyl-4-methylphenol)
2,2'-Methylen-bis-(6-tert.butyl-4-ethylphenol)
2,2'-Methylen-bis-[4-methyl-6-(α-methylcyclohexyl)-phenol]
2,2'-Methylen-bis-(4-methyl-6-cyclohexylphenol)
2,2'-Methylen-bis-(6-nonyl-4-methylphenol)
2,2'-Methylen-bis-(4,6-di-tert.butylphenol)
2,2'-Ethyliden-bis-(4,6-di-tert.butylphenol)
2,2'-Ethyliden-bis-(6-tert.butyl-4-isobutylphenol)
2,2'-Methylen-bis-[6-(α-methylbenzyl)-4-nonylphenol]
2,2'-Methlyen-bis-[6-(α,α-dimethylbenzyl)-4-nonylphenol]
4,4'-Methylen-bis-(2,6-di-tert.butylphenol)
4,4'-Methylen-bis-(6-tert.butyl-2-methylphenol)
1,1-Bis-(5-tert.butyl-4-hydroxy-2-methylphenyl)-butan
2,6-Di-(3-tert.butyl-5-methyl-2-hydroxybenzyl)-4-methylphenol
1,1,3-Tris-(5-tert.butyl-4-hydroxy-2-methylphenyl)-butan
1,1-Bis-(5-tert.butyl-4-hydroxy-2-methylphenyl)-3-n-dodecylmercaptobutan
Ethylenglycol-bis-[3,3-bis-(3'-tert.butyl-4'-hydroxyphenyl)-butyrat]
Di-(3-tert.butyl-4-hydroxy-5-methylphenyl)-dicyclopentadien
Di-[2-(3'-tert.butyl-2'-hydroxy-5'-methyl-benzyl)-6-tert.butyl-4-methyl-phenyl]-terephthalat.

1.5 Benzylverbindungen

1,3,5-Tri-(3,5-di-tert.butyl-4-hydroxybenzyl)-2,4,6-trimethylbenzol
Di-(3,5-di-tert.butyl-4-hydroxybenzyl)-sulfid
3,5-di-tert.butyl-4-hydroxybenzyl-mercaptoessigsäure-isooctylester
Bis-(4-tert.butyl-3-hydroxy-2,6-dimethylbenzyl)dithiol-terephthalat
1,3,5-Tris-(3,5-di-tert.butyl-4-hydroxybenzyl)-isocyanurat
1,3,5-Tris-(4-tert.butyl-3-hydroxy-2,6-dimethylbenzyl)-isocyanurat
3,5-Di-tert.butyl-4-hydroxybenzyl-phosphonsäure-dioctadecylester
3,5-Di-tert.butyl-4-hydroxybenzyl-phosphonsäure-monoethylester, Calcium-salz.

1.6. Acylaminophenole

4-Hydroxy-laurinsäureanilid
4-Hydroxy-stearinsäureanilid
2,4-Bis-octylmercapto-6-(3,5-di-tert.butyl-4-hydroxyanilino)-s-triazin
N-(3,5-di-tert.butyl-4-hydroxyphenyl)-carbaminsäureoctylester.

1.7. Ester der β-(3,5-Di-tert.butyl-4-hydroxyphenyl)-propionsäure
mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.8. Ester der β(5-tert.butyl-4-hydroxy-3-methylphenyl)-propionsäure
mit ein- oder mehrwertigen Alkoholen, wie z. B. mit

| | |
|---|---|
| Methanol | Diethylenglycol |
| Octadecanol | Triethylenglycol |
| 1,6-Hexandiol | Pentaerythrit |
| Neopentylglycol | Tris-hydroxyethyl-isocyanurat |
| Thiodiethylenglycol | Di-hydroxyethyl-oxalsäurediamid |

1.9. Amide der β-(3,5-Di-tert.butyl-4-hydroxyphenyl)-propionsäure, wie z. B.
N,N'-Di-(3,5-di-tert.butyl-4-hydroxyphenylpropionyl)-hexamethylendiamin
N,N'-Di-(3,5-di-tert.butyl-4-hydroxyphenylpropionyl)-trimethylendiamin
N,N'-Di-(3,5-di-tert.butyl-4-hydroxyphenylpropionyl)-hydrazin

**2. UV-Absorber und Lichtschutzmittel**

2.1. 2-(2'-Hydroxyphenyl)-benztriazole, wie z. B. das 5'-Methyl-, 3',5'-Di-tert.butyl-, 5'-Tert.butyl-, 5'-(1,1,3,3-Tetramethyl-butyl)-, 5-Chlor-3',5'-di-tert.butyl-, 5-Chlor-3'-tert.butyl-5'-methyl-, 3'-sec.Butyl-5'-tert.butyl, 4'-Octoxy-, 3',5'-Di-tert.amyl-, 3',5'-Bis-(α,α-dimethylbenzyl)-Derivat.

2.2. 2-Hydyroxybenzophenone, wie z. B. das 4-Hydroxy-, 4-Methoxy-, 4-Octoxy-, 4-Decyloxy-, 4-Dodecyloxy-, 4-Benzyloxy-, 4,2',4'-Trihydroxy-, 2'-Hydroxy-4,4'-dimethoxy-Derivat.

2.3. Ester von gegebenenfalls substituierten Benzoesäuren, wie z. B. 4-Tert.butyl-phenylsalicylat, Phenylsalicylat, Octylphenylsalicylat, Dibenzoylresorcin, Bis-(4-tert.butylbenzoyl)-resorcin, Benzoylresorcin, 3,5-Di-tert.butyl-4-hydroxybenzoesäure-2,4-di-tert.butylphenylester, 3,5-Di-tert.butyl-4-hydroxybenzoesäure hexadecylester.

2.4. Acrylate, wie z. B. α-Cyan-β,β-diphenylacrylsäure-ethylester bzw. -isooctylester, α-Carbomethoxy-zimtsäuremethylester, α-Cyano-β-methyl-p-methoxy-zimtsäuremethylester bzw. -butylester, α-Carbomethoxy-p-methoxy-zimtsäure-methylester, N-(β-Carbomethoxy-β-cyanovinyl)-2-methyl-indolin.

2.5. Nickelverbindungen, wie z. B. Nickelkomplexe des 2,2'-Thio-bis-[4-(1,1,3,3-tetramethylbutyl)-phenols], wie der 1 : 1- oder der 1 : 2-Komplex, gegebenenfalls mit zusätzlichen Liganden wie n-Butylamin, Triethanolamin oder N-Cyclohexyl-diethanolamin, Nickeldibutyldithiocarbamat, Nickelsalze von 4-Hydroxy-3,5-di-tert.butylbenzylphosphonsäure-monoalkylestern wie vom Methyl- oder Ethylester, Nickelkomplexe von Ketoximen wie von 2-Hydroxy-4-methyl-phenyl-undecylketonoxim, Nickelkomplexe des 1-Phenyl-4-lauroyl-5-hydroxy-pyrazols, gegebenenfalls mit zusätzlichen Liganden.

2.6. Sterisch gehinderte Amine, wie z. B. Bis-(2,2,6,6-tetramethylpiperidyl)-sebacat
Bis-(1,2,2,6,6-pentamethylpiperidyl)-sebacat n-Butyl-3,5-di-tert.butyl-4-hydroxybenzyl-malonsäure-bis(1,2 2,6,6-pentamethylpiperidyl)-ester, Kondensationsprodukt aus 1-Hydroxyethyl-2,2,6,6-Tetramethyl-4-hydroxypiperidin und Bernsteinsäure, Kondensationsprodukt aus N,N'-(2,2,6,6-Tetramethylpiperidyl)-hexamethylendiamin und 4-tert.octylamino-2,6-dichlor-1,3,5-s-triazin,
Tris-(2,2,6,6-tetramethylpiperidyl)-nitrilotriacetat, Tetrakis-(2,2,6,6-tetramethyl-4-piperidyl)-1,2,3,4-butantetracarbonsäure,
1,1'-(1,2-ethandiyl)-bis-(3,3,5,5-tetramethyl-piperazinon).

2.7. Oxalsäurediamide, wie z. B. 4,4'-Di-octyloxy-oxanilid, 2,2'-Di-octyloxy-5,5'-di-tert.butyl-oxanilid, 2,2'-Di-dodecyloxy-5,5'-di-tert.butyl-oxanilid, 2-Ethoxy-2'-ethyl-oxanilid, N,N'-Bis-(3-dimethylaminopropyl)-oxalamid, 2-Ethoxy-5-tert.butyl-2'-ethyl-oxanilid und dessen Gemisch mit 2-Ethoxy-2'-ethyl-5,4'-di-tert.butyl-oxanilid, Gemische von ortho- und para-Methoxy- sowie von o- und p-Ethoxy-di-substituierte oxaniliden.

3. Metalldesaktivatoren, wie z. B. N,N'-Diphenyloxalsäurediamid, N-Salicylal-N'-salicyloylhydrazin, N,N'-Bis-salicyloylhydrazin, N,N'-Bis-(3,5-di-tert.butyl-4-hydroxyphenylpropionyl)-hydrazin, 3-Salicyloylamino-1,2,4-triazol, Bis-benzyliden-oxalsäuredihydrazid.

4. Phosphite und Phosphonite, wie z. B. Triphenylphosphit, Diphenylalkylphosphite, Phenyldialkylphosphite, Tri-(nonylphenyl)-phosphit, Trilaurylphosphit, Trioctadecylphosphit, Distearyl-pentaerythritdiphosphit, Tris-(2,4-di-tert.butylphenyl)-phosphit, Diisodecylpentaerythrit-diphosphit, Di-(2,4-di-tert.butylphenyl)-pentaerythritdiphosphit, Tristearyl-sorbit-triphosphit, Tetrakis-(2,4-di-tert.butylphenyl)-4,4'-biphenylen-diphosphonit.

5. Peroxidzerstörende Verbindungen, wie z. B. Ester der β-Thio-dipropionsäure, beispielsweise der Lauryl-, Stearyl-, Myristyl- oder Tridecylester, Mercaptobenzimidazol, das Zinksalz des 2-Mercaptobenzimidazols, Zinkdibutyl-dithiocarbamat, Dioctadecyldisulfid, Pentaerythrit-tetrakis-(β-dodecylmercapto)-propionat.

6. Polyamidstabilisatoren, wie z. B. Kupfersalze in Kombination mit Jodiden und/oder Phosphorverbindungen und Salze des zweiwertigen Mangans.

7. Basische Co-Stabilisatoren, wie z. B. Melamin, Polyvinylpyrrolidon, Dicyandiamid, Triallylcyanurat, Harnstoff-Derivate, Hydrazin-Derivate, Amine, Polyamide, Polyurethane, Alkali- und Erdalkalisalze höherer Fettsäuren, beispielsweise Ca-Stearat, Zn-Stearat, Mg-Stearat, Na-Ricinoleat, K-Palmitat, Antimonbrenzcatechinat oder Zinnbrenzcatechinat.

8. Nukleierungsmittel, wie z. B. 4-tert.Butylbenzoesäure, Adipinsäure, Diphenylessigsäure.

9. Füllstoffe und Verstärkungsmittel, wie z. B. Calciumcarbonat, Silikate, Glasfasern, Asbest, Talk, Kaolin, Glimmer, Bariumsulfat, Metalloxide und -hydroxide, Russ, Graphit.

10. Sonstige Zusätze, wie z. B. Weichmacher, Gleitmittel, Emulgetoren, Pigmente, optische Aufheller, Flammschutzmittel, Antistatika, Treibmittel.

Die folgenden Beispiele erläutern die vorliegende Erfindung näher.

**Beispiel 1:**

In 100 ml Methylenchlorid werden bei 0 - 5°C 43,7 g (0,1 Mol) 3-(2H-Benzotriazol-2-yl)-4-hydroxy-5-tert.butyl-benzolpropansäure-(2-hydroxy) cyclohexylester und 13,3 g (0,105 Mol) Chlorpropionsäurechlorid gelöst. Bei dieser Temperatur lässt man langsam 21,1 g (0,21 Mol) Triethylamin zutropfen. Es bildet sich sofort ein Niederschlag von Triethylaminhydrochlorid. Nach dem Zutropfen lässt man ca. 30 Min. kalt ausrühren und danach die Temperatur auf 20 - 25°C ansteigen. Bei dieser Temperatur lässt man noch ca. 6 Std. ausrühren. Danach filtriert nan die Lösung und wäscht sie mit verdünnter Natriumcarbonatlösung. Die organische Phase wird eingedampft und das bräunliche Harz aus Hexan auskristallisiert. Man erhält 34,4 g (70,1 %) 3-(2H-Benzotriazol-2-yl)-4-hydroxy-5-tert.butyl-benzolpropansäure-(2-acrylyloxy)-cyclohexylester als weisses Kristallisat. Smp. 82 - 84°C.

**Beispiel 2:**

Verwendet man 3-(5-chlor-2H-benzotriazol-2-yl)-4-hydroxy-5-tert.butyl-benzolpropansäure-(2-hydroxy)-cyclohexylester und verfährt im übrigen genau gleich, wie in Beispiel 1 beschrieben, so erhält man 3-(5-Chlor-2H-benzotriazol-2-yl)-4-hydroxy-5-tert.butylbenzolpropansäure-(2-acrylyloxy)-cyclohexylester als weisses Kristallisat. Smp. 109 - 110°C.

**Beispiel 3:**

Verwendet man N-(2-Hydroxyethyl)-3-(2H-benzotriazol-2-yl)-4-hydroxy-5-tert.butyl-benzolpropanamid und verfährt wie in Beispiel 1 beschrieben, so erhält man N-(2-Acrylyloxyethyl)-3-(2H-benzotriazol-2-yl)-4-hydroxy-5-tert.butyl-benzolpropanamid als wiesses Kristallat. Smp. 80 - 83°C.

**Beispiel 4:**

Verwendet man N-(2-Hydroxyethyl)-3-(5-chlor-2H-benzotriazol-2-yl)-4-hydroxy-5-tert.butyl-benzolpropanamid, so erhält man bei wiederum gleichem Verfahren N-(2-Acrlyloxyethyl)-3-(5-chlor-2H-benzotriazol-2-yl)-4-hydroxy-5-tert.butyl-benzolpropanamid als weisses Kristallisat. Smp. 106 - 108°C.

**Beispiel 5:**

Verwendet man N-(3-Hydroxypropyl)-3-(2H-benzotriazol-2-yl)-4-hydroxy-5-tert.butyl-benzolpropanamid und verfährt analog wie in Beispiel 1 beschrieben, so erhält man N-(3-Acrylyloxypropyl)-3-(2H-benzotriazol-2-yl)-4-hydroxy-5-tert.butyl-benzolpropanamid als weisses Kristallisato, das aus Acetonitril umkristallisiert wird. Smp. 87 - 89°C.

**Beispiel 6:**

Verwendet man 3-(2H-Benzotriazol-2-yl)-4-hydroxy-5-tert.butyl-benzolpropansäure-(2-hydroxy)-propylester und verfährt genau gleich wie in Beispiel 1 beschrieben so erhält man 3-(2H-Benzotriazol-2-yl)-4-hydroxy-5-tert.butyl-benzolpropansäure-(2-acrylyloxy)-propylester als harzförmiges Produkt.

| | | |
|---|---|---|
| Elementanalyse: Ber.: | C = 66,50 % | Gef.: C = 66,51 % |
| | H = 6,47 % | H = 6,62 % |
| | N = 9,31 % | N = 9,27 % |

**Beispiel 7:**

Verwendet man 3-(2H-Benzotriazol-2-yl)-4-hydroxy-5-tert.butylbenzolpropansäure-(2-hydroxy)-butylester und verfährt genau gleich wie in Beispiel 1 beschrieben, so erhält man 3-(2H-Benzotriazol-2-yl)-4-hydroxy-5-tert.butylbenzolpropansäure-(2-acrylyloxy)-butylester als harzförmiges Produkt.

| | | |
|---|---|---|
| Elementanalyse: Ber.: | C = 67,08 % | Gef.: C = 67,28 % |
| | H = 6,71 % | H = 6,68 % |
| | N = 9,03 % | N = 9,07 % |

**Beispiel 8:**

Verwendet man 3-(2H-Benzotriazol-2-yl)-4-hydroxy-5-tert.butyl-benzolpropansäure-(2-hydroxy-2-phenyl)-äthylester und verfährt im übrigen genau gleich, wie in Beispiel 1 beschrieben, so erhält man 3-(2H-Benzotriazol-2-yl)-4-hydroxy-5-tert.butylbenzolpropansäure-(2-acrylyl-2-phenyl)-äthylester, als weisses Kristallisat, das aus Ligroin umkristallisierbar ist. Smp. 89 - 91° C.

**Beispiel 9:**

Verwendet man 3-(2H-Benzotriazol-2-yl)-4-hydroxy-5-tert.butyl-benzolpropansäure-(2-hydroxy-3-phenoxy)-propylester und verfährt im übrigen genau gleich, wie in Beispiel 1 beschrieben, so erhält man 3-(2H-Benzotriazol-2-yl)-4-hydroxy-5-tert.butyl-benzolpropansäure-(2-acrylyl-3-phenoxy)-propylester.

**Beispiel 10:**

In 300 ml Toluol werden 38.2 g N-(2-Hydroxyethyl)-3-(2H-benzotriazol-2-yl)-4-hydroxy-5-tert.butyl-benzolpropanamid mit 21.6 g Maleinsäuredimethylester gelöst. Man erhitzt die Lösung auf Rückfluss und gibt 1.24 g Dibutylzinnoxid als Katalysator hinzu. Darauf wird während 5 Stunden ein Gemisch Methanol/Toluol abdestilliert und dabei reines Toluol laufend durch den Tropftrichter wieder hinzugegeben. Mit der Zeit fällt ein beiger Niederschlag aus. Nach 5 Stunden Reaktionszeit filtriert man von diesem Niederschlag ab und dampft das orangefarbene Filtrat ein. Man erhält ein bräunliches Öl, das über Kieselgel mit $CH_2Cl_2$/-Essigsäureethylester = 9 : 1 chronatographiert wird. Die Produktefraktionen werden vereinigt und eingedampft. Der Rückstand wird aus Hexan und wenig Toluol umkristallisiert. Man erhält 26.3 g N-(2-(4-Methoxy-1,4-dioxo-cis-but-2-en-1-yloxy)-ethyl)-3-(2H-benzotriazol-2-yl)-4-hydroxy-5-tert.butyl-benzol-propanamid als weisses Kristallisat mit Smp. 72 - 75° C.

**Beispiel 11:**

<u>Bewitterungsresultate von Acrylharzfilmen mit einpolimerisierbaren UVA</u>

<u>Formulierung:</u> 82 Teile eines Polyesteracrylates (Ebecryl 584 Fa. UCB)
15 Teile Dicyclopentenyloxyäthylacrylat (Rohm + Haas)
3 Teile eines Verlaufshilfsmittel (Byk 300)
1,5 Teile Additiv

werden während 5 Minuten in einem Disolver homogenisiert. Die UV-Absorber werden zugegeben und nochmals während 5 Minuten im Disolver bei 45 - 50° C gemischt.

**Applikation:**

Auf ein vorgrundiertes und mit silbermetallic Lack überzogenes Al-Blech, werden elektromotorisch mit einer Spiralrakel die Klarlacke so aufgezogen, dass Trockenfilme von 40 ± 3 µm erzielt erzielt werden. Diese werden anschliessend in einer Elektronenbestrahlungs-Laboranlage mit 6 M rad ausgehärtet. Die so gehärteten Filme werden anschliessend in einem QUV-Belichtungsgerät mit folgendem Zyklus belichtet:

4h UV Licht bei 60°C
4h ohne Belichtung bei 50°C
usw.

Alle 100h werden die Proben auf Glanzerhaltung und Rissbildung überprüft.

**Resultate**

| | Glanzerhaltung in % | Sichtbare Rissbildung nach |
|---|---|---|
| ohne Additiv | 40 | 300h |
| Additiv Beispiel 5 | > 70 | > 500h |
| Additiv Beispiel 6 | > 70 | > 500h |
| Additiv Beispiel 7 | > 70 | > 500h |

**Patentansprüche**

1. Verbindungen der Formel I

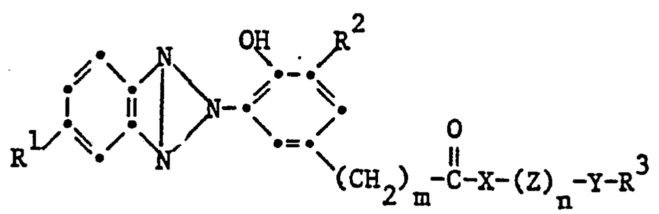

(I),

worin

X -O- oder $-N(R^4)-$ bedeutet, und
Y -O- oder $-N(R^5)-$ ist, und
Z $C_2$-$C_{12}$ Alkylen, durch ein bis drei $-N(R^4)$-Gruppen und/oder Sauerstoff-Atome unterbrochenes $C_4$-$C_{12}$ Alkylen, durch eine Hydroxygruppe substituiertes $C_3$-$C_{12}$ Alkylen, Butenylen, Butinylen, Cyclohexylen oder Phenylen bedeutet, und
m eine Zahl 0, 1 oder 2 ist und
n 1, und falls X und Y $-N(R^4)-$ bzw. $-N(R^5)$ sind, auch O bedeutet, und
$R^1$ Wasserstoff, Chlor, $C_1$-$C_4$ Alkyl oder $C_1$-$C_4$ Alkoxy ist, und
$R^2$ Wasserstoff oder $C_1$-$C_6$ Alkyl bedeutet, und
$R^3$ eine Gruppe $-C(O)-C(R^6) = C(H)R^7$ ist, oder falls Y $-N(R^5)-$ ist, zusammen mit $R^5$ eine Gruppe $-C(O)-CH = CH-C(O)-$ bildet, wobei $R^6$ Wasserstoff oder Methyl ist, und $R^7$ Wasserstoff, Methyl oder $-C(O)-X-R^8$ bedeutet, wobei $R^8$ Wasserstoff, $C_1$-$C_{12}$ Alkyl oder eine Gruppe der Formel II bedeutet

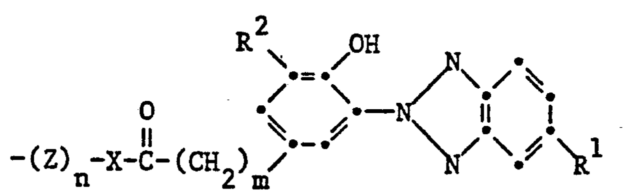

(II),

worin die Symbole $R^1$, $R^2$ X, Z, m und n die oben angegebene Bedeutung haben, und
$R^4$ und $R^5$ unabhängig voneinander Wasserstoff, $C_1$-$C_{12}$ Alkyl, durch 1 bis 3 Sauerstoffatome unterbrochenes $C_3$-$C_{12}$ Alkyl, Cyclohexyl oder $C_7$-$C_{11}$ Aralkyl bedeuten, und $R^4$ ferner zusammen mit $R^5$, falls Z Ethylen ist auch Ethylen bilden.

2. Verbindungen gemäss Anspruch 1 der Formel I, worin $R^3$ eine Gruppe $-C(O)-C(R^6) = C(H)R^7$ bedeutet.

3. Verbindungen gemäss Anspruch 2, wobei $R^6$ und $R^7$ unabhängig voneinander Wasserstoff oder Methyl

sind.

4. Verbindungen gemäss Anspruch 1 der Formel I, worin $R^1$ Wasserstoff oder Chlor ist.

5. Verbindungen gemäss Anspruch 1 der Formel I, worin m 2 bedeutet.

6. Verbindungen gemäss Anspruch 1 der Formel I, worin $R^2$ tert.Butyl ist.

7. Verbindungen gemäss Anspruch 1 der Formel I, worin X -O- oder $-N(R^4)-$ und Y -O- oder $-N(R^5)-$ bedeutet, Z $C_2$-$C_6$ Alkylen, 2-Hydroxypropylen oder Cyclohexylen ist, m 2 und n 1 bedeutet, und $R^1$ Wasserstoff oder Chlor ist, und $R^2$ Wasserstoff oder $C_1$-$C_8$ Alkyl bedeutet und $R^3$ eine Gruppe $-C(O)-C(R^6) = C(H)R^7$ ist, wobei $R^6$ und $R^7$ unabhängig voneinander Wasserstoff oder Methyl sind, und $R^3$ falls Y $-N(R^5)-$ ist, zusammen mit $R^5$ eine Gruppe -C(O)-CH= CH-C(O)- bildet, und $R^4$ und $R^5$ unabhängig voneinander Wasserstoff oder $C_1$-$C_4$ Alkyl sind.

8. Verwendung der Verbindungen der Formel I gemäss Anspruch 1 zum Schützen von Polymeren gegen lichtinduzierten Abbau.

9. Copolymere erhältlich aus ethylenisch ungesättigten Verbindungen und Verbindungen der Formel I gemäss Anspruch 1.

**Claims**

1. A compound of the formula I

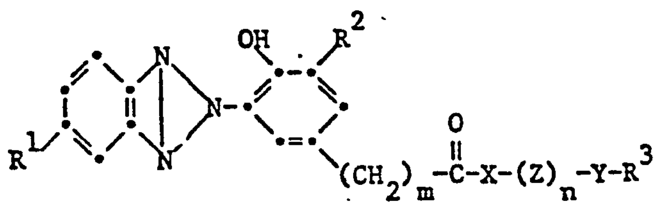

(I)

wherein

X is -O- or $-N(R^4)-$, and

Y is -O- or $-N(R^5)-$, and

Z is $C_2$-$C_{12}$alkylene, $C_4$-$C_{12}$alkylene interrupted by one to three $-N(R^4)$-groups and/or oxygen atoms, or is $C_3$-$C^{12}$ alkylene, butenylene, butynylene, cyclohexylene or phenylene, each substituted by a hydroxyl group and

m is a number 0, 1 or 2,

n is 1 and when X and Y are $-N(R^4)-$ and $-N(R^5)-$, respectively, is also O, and

$R^1$ is hydrogen, chlorine, $C_1$-$C_4$alkyl or $C^1$-$C_4$ alkoxy and

$R^2$ is hydrogen or $C_1$-$C_8$alkyl and

$R^3$ is a group $-C(O)-C(R^6) = C(H)R^7$ or, when Y is $-N(R^5)-$, forms together with $R^5$ a group -C(O)-CH = CH-C(O)-, wherein $R^6$ is hydrogen or methyl, and $R^7$ is hydrogen methyl or $-C(O)-X-R^8$, wherein $R^8$ is hydrogen $C_1$-$C_{12}$alkyl or a group of the formula II

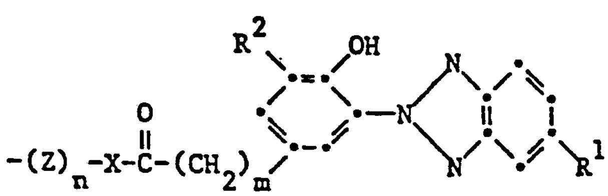

(II),

wherein the symbols $R^1$, $R^2$, X, Z, m and n are as defined above, and $R^4$ and $R^5$ independently of one another are hydrogen, $C_1$-$C_{12}$alkyl, $C_3$-$C_{12}$alkyl interrupted by 1 to 3 oxygen atoms, or is cyclohexyl or $C_7$-$C_{11}$aralkyl, and $R^4$ together with $R^5$ in the case where Z is ethylene, also form ethylene.

2. A compound according to claim 1 of the formula I wherein $R^3$ is a group $-C(O)-C(R^6) = C(H)R^7$.

3. A compound according to claim 2 wherein $R^6$ and $R^7$ independently of one another are hydrogen or methyl.

4. A compound according to claim 1 of the formula I, wherein $R^1$ is hydrogen or chlorine.

5. A compound according to claim 1 of the formula I wherein m is 2.

6. A compound according to claim 1 of the formula I, wherein $R^2$ is tertbutyl.

7. A compound according to claim 1 of the formula I, wherein X is -O- or $-N(R^4)-$ and Y is -O- or $-N(R^5)-$, Z is $C_2$-$C_6$alkylene, 2-hydroxypropylene or cyclohexylene, m is 2 and n is 1, $R^1$ is hydrogen or chlorine, $R^2$ is hydrogen or $C_1$-$C_8$alkyl, and $R^3$ is a group $-C(O)-C(R^6) = C(H)R^7$, wherein $R^6$ and $R^7$ independently of one another are hydrogen or methyl, and $R^3$, when Y is $-N(R^5)-$, forms together with $R^5$ a group -C(O)-CH = CH-C(O)-, and $R^4$ and $R^5$ independently of one another are hydrogen or $C_1$-$C_4$alkyl.

8. Use of a compound of the formula I according to claim 1 for the protection of polymers against light-induced degradation.

9. Copolymers obtainable from ethylenically unsaturated compounds and compounds of the formula I according to claim 1.

**Revendications**

1. Composés de formule I

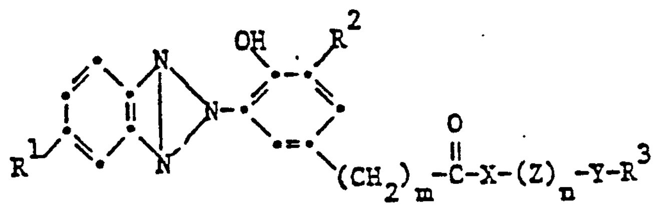

(I),

dans laquelle

X représente -O- ou un groupe $-N(R^4)-$, et

Y représente -O- ou un groupe $-N(R^5)-$ et

Z représente un groupe alkylène en $C_2$ à $C_{12}$ un groupe alkylène en $C_4$ à $C_{12}$ interrompu par un à trois groupes $-N(R^4)-$ et/ou atome(s) d'oxygène, un groupe alkylène en $C_3$ à $C_{12}$, buténylène, butynylène, cyclohexylène ou phénylène, substitué par un groupe hydroxyle, et

m est un nombre valant 0, 1 ou 2,

n vaut 1 et, si X et Y représentent $-N(R^4)-$ ou $-N(R^5)$, n peut aussi être nul, et

$R^1$ représente un atome d'hydrogène ou de chlore ou un groupe alkyle en $C_1$ à $C_4$ ou alcoxy en $C_1$ à $C_4$, et

$R^2$ représente un atome d'hydrogène ou un groupe alkyle en $C_1$ à $C_8$, et

$R^3$ représente un groupe $-C(O)-C(R^6) = C(H)R^7$, ou bien, si Y représente $-N(R^5)-$, $R^3$ forme avec $R^5$ un groupe $-C(O)-CH=CH-C(O)-$, où $R^6$ représente un atome d'hydrogène ou un groupe méthyle, et $R^7$ représente un atome d'hydrogène, un groupe méthyle ou $-C(O)-X-R^8$, où $R^8$ représente un atome d'hydrogène, un groupe alkyle en $C_1$ à $C_{12}$ ou un groupe de formule

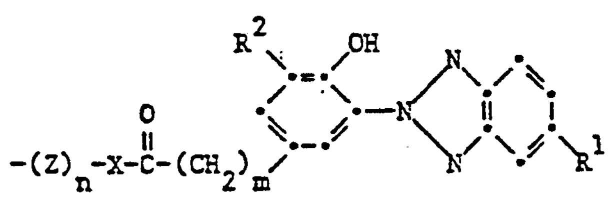

(II),

dans laquelle les symboles $R^1$, $R^2$, X, Z, m et n ont le sens indiqué ci-dessus et,

$R^4$ et $R^5$ représentent indépendamment l'un de l'autre un atome d'hydrogène un groupe alkyle en $C_1$ à $C_{12}$, un groupe alkyle en $C_3$ à $C_{12}$, cyclohexyle ou aralkyle en $C_7$ à $C_{11}$ interrompu par 1 à 3 atomes d'oxygène, et $R^4$ peut en outre former avec $R^5$ également un groupe éthylène si Z représente un groupe éthyléne.

2. Composés selon la revendication 1, de formule I, dans laquelle $R^3$ représente un groupe $-C(O)-C(R^6)=C(H)R^7$.

3. Composés selon la revendication 2, dans lesquels $R^6$ et $R^7$ représentent, indépendamment l'un de l'autre, un atome d'hydrogène ou un groupe méthyle.

4. Composé selon la revendication 1, de formule I, dans laquelle $R^1$ représente un atome d'hydrogène ou de chlore.

5. Composés selon la revendication 1, de formule I, dans laquelle m vaut 2.

6. Composés selon la revendication 1, de formule I, dans laquelle $R^2$ est un groupe tertio-butyle.

7. Composés selon la revendication 1, de formule I, dans laquelle X représente -O- ou $-N(R^4)-$ et Y représente -O- ou $-N(R^5)-$, Z représente un groupe alkylène en $C_2$ à $C_6$, hydroxy-2 propylène ou cyclohexylène, m vaut 2 et n vaut 1, et $R^1$ représente un atome d'hydrogène ou de chlore, $R^2$ représente un atome d'hydrogène ou un groupe alkyle en $C_1$ à $C_8$, et $R^3$ représente un groupe $-C(O)-C(R^6) = C(H)R^7$, où $R^6$ et $R^7$ représentent, indépendamment l'un de l'autre, un atome d'hydrogène ou un groupe méthyle et, si Y représente $-N(R^5)-$, $R^3$ forme avec $R^5$ un groupe $-C(O)-CH=CH-C(O)-$, et $R^4$ et $R^5$ représentent, indépendamment l'un de l'autre, un atome d'hydrogène ou un groupe alkyle en $C_1$ à $C_4$.

8. Utilisation des composés de formule I selon la revendication 1, pour protéger des polymères contre une

dégradation provoquée par la lumière.

9. Copolymères pouvant être obtenus à partir de composés à insaturation éthylénique et de composés de formule I selon la revendication 1.